# EUROPEAN PATENT APPLICATION

(11) **EP 1 728 511 A1**
(43) Date of publication of application: **06.12.2006**
(21) Application number: 05291172.4
(22) Date of filing: 31.05.2005
(51) Int. Cl.: A61K 31/43, A61P 25/00

(54) **New use of phenethicillin and salts thereof.**

(71) Applicant: Faust Pharmaceuticals, 67400 Illkirch (FR)
(72) Inventor: Neuville, Pascal, 67610 La Wantzenau (FR); Fintz, Anne-Claire, 67201 Eckbolsheim (FR)

(57) **Abstract**

The present invention provides the use of phenethicillin and salts thereof for the manufacture of a drug especially useful in the treatment and/or prophylaxis of neurological diseases and conditions such as chronic neurodegenerative disorders (dementia of the Alzheimer's type, amyotrophic lateral sclerosis, Parkinson's disease or Huntington's disease) and acute neurodegenerative disorders (stroke or traumatic brain injury).

## Description

The present invention provides the use of phenethicillin and salts thereof for the manufacture of a drug especially useful in the treatment and/or prophylaxis of neurological diseases and conditions such as chronic neurodegenerative disorders (dementia of the Alzheimer's type, amyotrophic lateral sclerosis, Parkinson's disease or Huntington's disease) and acute neurodegenerative disorders (stroke or traumatic brain injury).

The term "neuroprotection" refers to interventions that produce enduring benefits by favourably influencing underlying ethiology or pathogenesis of neurodegenerative diseases and conditions, and therefore forestalling onset of illness or clinical decline (Shoulson I (1998) Neuroprotection Science 282, 1072-1074).
From a molecular or a genetic point of view, but also from a clinical point of view, degeneration of neurons results from heterogeneous causes. The numerous recent insights into the genetic cause and/or ethiology of many neurodegenerative diseases and conditions have not yet provided any clue to effective therapies. To characterize novel therapeutic properties of certain compounds, different from those a drug was initially developed for, is one among several neuroprotective approaches. The systematic screening of marketed compounds in neurodegenerative/neuroprotective experimental paradigms may contribute to identify such compounds. Previous clinical and safety data concerning these compounds will contribute to rapidly design new clinical developments for the treatment and/or prophylaxis of neurodegenerative diseases and conditions.

Penicillin, derived from the *Penicillium* soil mold, is one of the earliest discovered and widely used antibiotic agents. Since the use of penicillin was considered as a medical miracle in 1940-1945, a considerable research effort was directed to the study of penicillin derivatives.

One of them, a methyl analogue of penicillin V, displays particularly interesting neuroprotective properties. This analogue called phenethicillin, or (2S,5R,6R)-3,3-Dimethyl-7-oxo-6-[(1-oxo-2-phenoxypropyl) amino]-4-thia-1-azabicyclo[3.2.0]heptane-2-carboxylic acid, is already known as an antibiotic under various tradenames among which Broxil® (Glaxo-Smithkline) or Syncillin® (Brystol Myers).

The inventors have surprisingly discovered that phenethicillin displays interesting neuroprotective properties. Consequently, phenethicillin is useful in the treatment of neurological diseases and conditions, such as epilepsy, including newborn, infantile, childhood and adult syndromes, partial (localization-related) and generalized epilepsies, with partial and generalized, convulsive and non-convulsive seizures, with and without impairment of consciousness, and status epilepticus; Dementias, including dementias of the Alzheimer's type (DAT), Pick's disease, vascular dementias, Lewy-body disease, dementias due to metabolic, toxic and deficiency diseases (including alcoholism, hypothyroidism, and vitamin B12 deficiency), AIDS-dementia complex, Creutzfeld-Jacob disease and atypical subacute spongiform encephalopathy; Parkinsonism and movement disorders, including multiple system atrophy, progressive supranuclear palsy, hepatolenticular degeneration, chorea (including Huntington's disease and hemiballismus), athetosis, dystonias (including spasmodic torticollis, occupational movement disorder, Gilles de la Tourette syndrome), tardive or drug induced dyskinesias, tremor and myoclonus; Motor neuron disease or amyotrophic lateral sclerosis (ALS); Other neurodegenerative and/or hereditary disorders of the nervous system, including spinocerebrellar degenerations such as Friedrich's ataxia and other hereditary cerebellar ataxias, predominantly spinal muscular atrophies, hereditary neuropathies, and phakomatoses; Disorders of the peripheral nervous system, including trigeminal neuralgia, facial nerve disorders, disorders of the other cranial nerves, nerve root and plexus disorders, mononeuritis such as carpal tunnel syndrome and sciatica, hereditary and idiopathic peripheral neuropathies, inflammatory and toxic neuropathies; Multiple sclerosis and other demyelinating diseases of the nervous system; Infantile cerebral palsy (spastic), monoplegic, paraplegic or tetraplegic; Hemiplegia and hemiparesis, flaccid or spastic, and other paralytic syndromes; Cerebrovascular disorders, including subarachnoid hemorrhage, intracerebral hemorrhage, occlusion and stenosis of precerebral arteries, occlusion of cerebral arteries including thrombosis and embolism, brain ischemia, stroke, transient ischemic attacks, atherosclerosis, cerebrovascular dementias, aneurysms, cerebral deficits due to cardiac bypass surgery and grafting; Migraine, including classical migraine and variants such as cluster headache; Headache; Myoneural disorders including myasthenia gravis, acute muscle spasms, myopathies including muscular dystrophies, mytotonias and familial periodic paralysis; Disorders of the eye and visual pathways , including retinal disorders, and visual disturbances,; Intracranial trauma/injury and their sequels; Trauma/injury to nerves and spinal cord and their sequels; Poisoning and toxic effects of nonmedicinal substances; Accidental poisoning by drugs, medicinal substances and biologicals acting on the central, peripheral and autonomic system; Neurological and psychiatric adverse effects of drugs, medicinal and biological substances; Disturbance of sphincter control and sexual function; Mental disorders usually diagnosed in infancy, childhood or adolescence, including : mental retardation, learning disorders, motor skill disorders, communication disorders, pervasive developmental disorders, attention deficit and disruptive behaviour disorders, feeding and eating disorders, TIC disorders, elimination disorders; Delirium and other cognitive disorders; Substance related disorders including: alcohol-related disorders, nicotine-related disorders, disorders related to cocaine, opioids, cannabis, hallucinogens and other drugs; Schizophrenia and other psychotic disorders; Mood disorders, including depressive disorders and bipolar disorders; Anxiety disorders, including panic disorders, phobias, obsessive-compulsive disorders, stress disorders, generalized anxiety disorders; Eating disorders, including anorexia and bulimia; Sleep disorders, including dyssomnias (insomnia, hypersomnia, narcolepsy, breathing related sleep disorder) and parasomnias; Medication-induced movement disorders (including neuroleptic-induced parkinsonism and tardive dyskinesia); Endocrine and metabolic diseases including diabetes, disorders of the endocrine glands, hypoglycaemia; Acute and chronic pain; Nausea and vomiting; Irritable bowel syndrome.

The present invention provides the use of phenethicillins of formula (I): 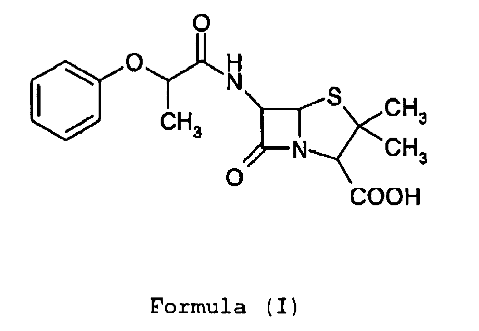 as well as pharmaceutically acceptable salt thereof, a prodrug of the compound or the salt, or a solvate or hydrate of the compound, the salt or the prodrug;
for the manufacture of a drug allowing neuroprotection, for the treatment and/or prophylaxis of neurological diseases and conditions.

The present invention includes salts of the compounds of formula (I). These salts can exist in conjunction with the acidic portion of the molecule and can exist as primary, secondary, tertiary, or quaternary ammonium, alkali metal, or alkaline earth metal salts. The alkali metal and alkaline earth metal salts are generally prepared by the reaction of the hydroxide form of the desired metal with a compound of formula (I)

It will be appreciated that the compounds of formulae (I) contain four asymmetric carbon atoms, three being in the 6-aminopenicillanic acid moiety and one being in the phenoxy-side chain. Accordingly, the compounds of the invention may exist in and be isolated in enantiomerically pure form or in racemic form or in a diastereosomeric mixture.

Among the preferred salts of the compound of formula (I) is (2S,5R,6R)-3,3-Dimethyl-7-oxo-6-[(1-oxo-2-phenoxy propyl)amino]-4-thia-1-azabicyclo[3.2.0]heptane-2-carboxylic acid monopotassium salt, or phenethicillin monopotassium salt of formula (II): 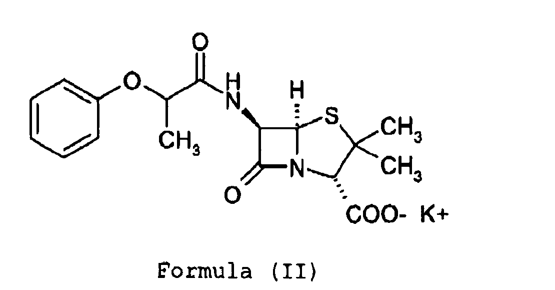 as well as a prodrug of the compound, or a solvate or hydrate of the compound or the prodrug.

Compound of formula (II) is known and commonly called phenethicillin potassium (Merck index, 13th edition (2001), 7303*).* It can be prepared from 6-aminopenicillanic acid (Perron et al., Antibiot. Ann. 1959-1960, 107; J. Am. Chem. Soc. 82, 3934 (1960); Glombitza, Ann. 673, 166 (1964)).

It will be appreciated that the compounds of formulae (I) contain four asymmetric carbon atoms, three being in the 6-aminopenicillanic acid moiety and one being in the phenoxy-side chain. Accordingly, the compounds of the invention may exist in and be isolated in enantiomerically pure form or in racemic form or in a diastereosomeric mixture.

In a preferred embodiment, the present invention provides the use of phenethicillin potassium of formula (II): 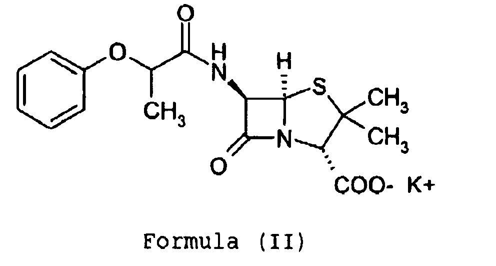 as well as a prodrug of the compound, or a solvate or hydrate of the compound or the prodrug;
for the manufacture of a drug allowing neuroprotection, for the treatment and/or prophylaxis of neurological diseases and conditions.

Such neurological diseases and conditions are selected from, but not limited to epilepsy, dementias (including dementias of the Alzheimer's type, vascular dementias, AIDS-dementia complex, etc...), parkinsonism and movement disorders (including Huntington's disease, dystonias, Gilles de la Tourette syndrome, dyskinesias etc...), motor neuron disease or amyotrophic lateral sclerosis (ALS), other neurodegenerative and/or hereditary disorders of the nervous system (including hereditary cerebellar ataxias and spinal muscular atrophies), disorders of the peripheral nervous system, including trigeminal neuralgia and peripheral neuropathies, multiple sclerosis and other demyelinating diseases of the nervous system, infantile cerebral palsy, spasticity, hemiplegia and hemiparesis, cerebrovascular disorders (including brain ischemia, stroke, transient ischemic attacks, atherosclerosis, etc...), headache, migraine, myoneural disorders (myasthenia gravis, acute muscle spasms, myopathies, etc...), disorders of the eye andvisual pathways, intracranial trauma/injury, trauma/injury to nerves and spinal cord, poisoning and toxic effects of nonmedicinal substances, accidental poisoning by drugs medicinal substances and biologicals, neurological and psychiatric adverse effects of drugs, medicinal and biological substances (including medication-induced movement disorders), disturbance of sphincter control and sexual function, mental disorders usually diagnosed in infancy, childhood or adolescence (including, attention deficit and disruptive behaviour disorders, autism, TIC disorders, etc ...), delirium and other cognitive disorders, substance related disorders (alcohol, nicotine, drugs), schizophrenia and other psychotic disorders, mood disorders (including depressive disorders and bipolar disorders), anxiety disorders, sexual disorders, eating disorders, sleep disorders, endocrine and metabolic diseases (diabetes, hypoglycaemia), acute and chronic pain; nausea and vomiting; irritable bowel syndrome.

According to the invention, the terms "treatment and/or prophylaxis" refer to a process that is intended to produce a beneficial change in the condition of a mammal, e.g., a human, often referred to as a patient. A beneficial change can, for example, include one or more of: restoration of function, reduction of symptoms, limitation or retardation of progression of a disease, disorder, or condition or prevention, limitation or retardation of deterioration of a patient's condition, disease or disorder, improvement of the patient's quality of life.

In another embodiment, the present invention provides pharmaceutical compositions comprising a pharmaceutically effective amount of a compound of formula (I), a pharmaceutically acceptable salt thereof, a prodrug of the compound or the salt, or a solvate or hydrate of the compound, the salt or the prodrug, in combination with a pharmaceutically acceptable carrier, diluent or excipient.

In yet another embodiment, the present invention provides pharmaceutical compositions comprising a pharmaceutically effective amount of a salt of formula (II), a prodrug thereof, or a solvate or hydrate of the the salt or the prodrug, in combination with a pharmaceutically acceptable carrier, diluent or excipient.

The pharmaceutical compositions are prepared by known procedures using well-known and readily available ingredients. In making the compositions of the present invention, the active ingredient will usually be mixed with a carrier, or diluted by a carrier, or enclosed with a carrier, and may be in the form of a capsule, sachet, paper, or other container. When the carrier serves as a diluent, it may be a solid, semi-solid, or liquid material which acts as a vehicle, excipient, or medium for the active ingredient. The compositions can be in the form of tablets, pills, powders, lozenges, sachets, cachets, elixirs, suspensions, emulsions, solutions, syrups, aerosols, ointments containing, for example up to 10% by weight of active compound, soft and hard gelatin capsules, suppositories, sterile injectable solutions, and sterile packaged powders.

Some examples of suitable carriers, excipients and diluents include lactose, dextrose, sucrose, sorbitol, mannitol, starches, gum, acacia, calcium phosphate, alginates, gelatin, calcium silicate, microcrystalline cellulose, water syrup, methyl cellulose, methyl and propyl hydrobenzoates, talc, magnesium stearate and mineral oil. In addition, the compositions can include lubricating agents, wetting agents, preservatives, solubilizers, stabilizers, emulsifiers, sweeteners, colorants, flavorants, salts for varying the osmotic pressure, buffers, masking agents and antioxidants. They can also contain still other therapeutically valuable substances. The compositions of the invention may be formulated so as to provide quick, sustained, or delayed release of the active ingredient after administration to the patient by employing procedures well known in the art.

The particular dose of compound of formulae (I) or (II), administered according to this invention shall be determined by the particular circumstances surrounding the case, including the compound administered, the route of administration, the particular condition being treated, and similar considerations. The compounds can be administered by a variety of routes including oral, rectal, transdermal, subcutaneous, intravenous, intramuscular, or intranasal routes. Additionally, the compound of the invention may be administered by continuous infusion. A typical daily dose shall contain from about 0,01 mg/kg to about 100 mg/kg of the active compound according to the invention. Preferably, daily doses will be about 0,05 mg/kg to about 50 mg/kg, more preferably from about 0,1 mg/kg to about 25 mg/kg.

The pharmaceutical compositions of the invention are particularly useful for the treatment of neurological diseases or conditions selected from epilepsy, dementias (including dementias of the Alzheimer's type, vascular dementias, AIDS-dementia complex, etc...), parkinsonism and movement disorders (including Huntington's disease, dystonias, Gilles de la Tourette syndrome, dyskinesias etc...), motor neuron disease or amyotrophic lateral sclerosis (ALS), other neurodegenerative and/or hereditary disorders of the nervous system (including hereditary cerebellar ataxias and spinal muscular atrophies), disorders of the peripheral nervous system, including trigeminal neuralgia and peripheral neuropathies, multiple sclerosis and other demyelinating diseases of the nervous system, infantile cerebral palsy, spasticity, hemiplegia and hemiparesis, cerebrovascular disorders (including brain ischemia, stroke, transient ischemic attacks, atherosclerosis, etc...), headache, migraine, myoneural disorders (myasthenia gravis, acute muscle spasms, myopathies, etc...), disorders of the eye and visual pathways, intracranial trauma/injury, trauma/injury to nerves and spinal cord, poisoning and toxic effects of nonmedicinal substances, accidental poisoning by drugs medicinal substances and biologicals, neurological and psychiatric adverse effects of drugs, medicinal and biological substances (including medication-induced movement disorders), disturbance of sphincter control and sexual function, mental disorders usually diagnosed in infancy, childhood or adolescence (including, attention deficit and disruptive behaviour disorders, autism, TIC disorders, etc ...), delirium and other cognitive disorders, substance related disorders (alcohol, nicotine, drugs), schizophrenia and other psychotic disorders, mood disorders (including depressive disorders and bipolar disorders), anxiety disorders, sexual disorders, eating disorders, sleep disorders, endocrine and metabolic diseases (diabetes, hypoglycaemia), acute and chronic pain; nausea and vomiting; irritable bowel syndrome.

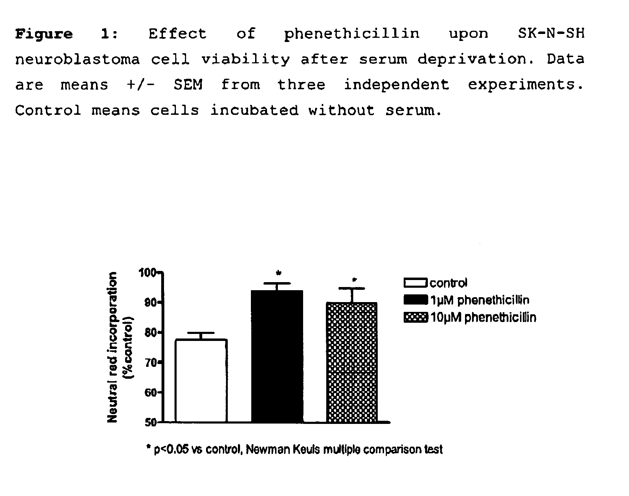

Other characteristics and advantages of the invention are given in the following examples in which reference is made to Figure 1 which represents the effect of phenethicillin upon SK-N-SH neuroblastoma cell viability after serum deprivation.

### Examples:

### Screening for neuroprotective drugs: In vitro analysis of neuroblastoma cell survival in serum deprivation assay

Protection of cell integrity and viability by survival factors is described in embryonic development as well as in cell interaction in mature system.

Serum provides survival factors (ex: growth factors) necessary to cell viability in culture conditions. According to the serum concentration it is possible to modulate the state of the cell viability. Total suppression of these components triggers cell death via apoptosis or necrosis. Causes of cell death could be oxidative stress and free radical accumulation, mitochondrial dysfunction or imbalanced calcium homeostasis.
Human SK-N-SH is a neuronal cell line usually cultured in serum containing medium. Serum withdrawal in the cell culture condition provides a cell-based assay allowing the study of neuroprotection and the screening for drugs capable of protecting cells from death or delaying cell death (Ba et al. (2003) The establishment of a reliable cytotoxic system with SK-N-SH neuroblastoma cell culture. J Neurosci. Methods 123:11-22, Green et al. (1997) 17α-oestradiol exerts neuroprotective effect on SK-N-SH cells. J Neurosci. 17(2):511-515)

### Cell culture

SK-N-SH cells (passage 35) were obtained from ECACC. They were routinely cultured in DMEM (PAA E15-810) containing 10% fetal calf serum (FCS), 1mM sodium pyruvate, and MEM non essential amino acids (L-alanine, 8.9; L-asparagine, 13.2; L-aspartic acid, 13.3; L-glutamic acid, 14.7 L-glycine, 7.5; L-proline , 11.5; L-serine, 10.5 mg/L) (PAA laboratories, Les Mureaux, France). Cells were cultured in monolayer in 175 cm² plastic flasks at 37°C and under 5% CO2, and 95% air. Medium was changed every 3 days and cells were passed once they reached approximately 80% confluence (every 3 days)

### Experimental paradigm

Cells grown in 10%FCS containing medium were seeded in 3%FCS containing DMEM at 10⁵cells/cm2 in 96-well plates. Cells were allowed to attach the plastic dishes 24h. After a PBS wash, cells were incubated either in DMEM without FCS (negative control) or in 3 % FCS containing DMEM (positive control) or in medium without FCS plus 1 or 10 µM of compound. Cell viability was assessed 48h later.

### Cell viability

The neutral red (NR) cytotoxicity assay procedure is a cell viability assay, based on the ability of viable cells to incorporate and bind neutral red, a supravital dye. Most cells will accumulate NR in lysosomes. The process requires intact membranes and active metabolism in the cell. Alterations of the cell surface or the sensitive lysosomal membrane lead to lysosomal fragility. Failure to take up NR therefore indicates that the cells have suffered damage.
The dye (3mg/mL) is allowed to be taken up by the cells one hour at 37°C, 5% CO₂, and is subsequently extracted by mixture of acetic acid and ethanol (1/50 V/V) and measured at 540 nm.

### Screening library

The library of compounds for this study was the Prestwick chemical library. The library is composed of 880 compounds in 96-well plates that also included control. The library is a unique collection of known bioactive compound (all off patent) selected for structural diversity, broad spectrum covering several therapeutic areas (from neuropsychiatry to cardiology, immunology, antiinflammatory, analgesia and more) and displaying known safety and bioavailability in humans. Over 85% of its compounds are marketed drugs.
Screening design consisted of testing once the complete chemical library at a final concentration of 1 and 10µM, to select the 50 compounds displaying the strongest "delta" (% viability of the culture treated with compound - % viability in negative control) and to test twice these 50 compounds.

### Results

Neuronal viability of compounds treated neuroblastoma cells was evaluated with the neutral red incorporation assay. Serum deprivation in cell cultures induced a loss of 26% in neutral red incorporation. Among the 50 compounds tested three times; phenethicillin displayed a reproducible effect by protecting this loss of about 6-16%.
In a dose range of 1-10µM, phenethicillin significantly delayed neuroblastoma cell death.

All publications and patent applications cited in this specification are herein incorporated by reference as if each individual publication or patent application was specifically and individually indicated to be incorporated by reference. Although the foregoing invention has been described in some detail by way of illustration and example for purposes of clarity of understanding, it will be readily apparent to those of ordinary skill in light of the teachings of this invention that certain changes and modifications may be made thereto without departing from the spirit or scope of the appended claims.

The invention has been described in an illustrative manner, and it is to be understood that the terminology which has been used is intended to be in the nature of words of description rather than of limitation. Obviously, many modifications and variations of the present invention are possible in light of the above teachings. It is therefore to be understood that within the scope of the appended claims, the invention may be practised otherwise than as specifically described. Accordingly, those skilled in the art will recognize, or able to ascertain using no more than routine experimentation, many equivalents to the specific embodiments of the invention described specifically herein. Such equivalents are intended to be encompassed in the scope of the following claims.

## Claims

1. Use of phenethicillins of formula (I): 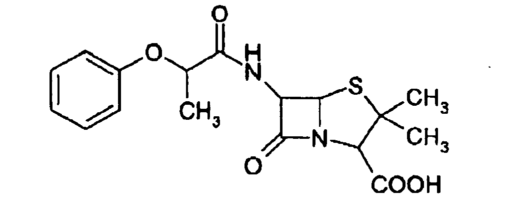 a pharmaceutically acceptable salt thereof, a prodrug of the compound or the salt, or a solvate or hydrate of the compound, the salt or the prodrug;
for the manufacture of a drug allowing neuroprotection for the treatment and/or prophylaxis of neurological diseases and conditions.

2. Use of phenethicillin potassium of formula (II): 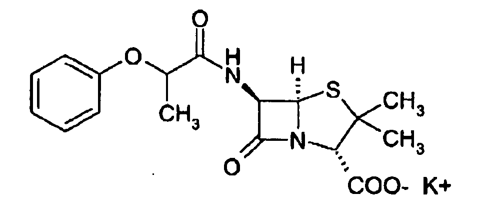 a pharmaceutically acceptable salt thereof, a prodrug of the compound or the salt, or a solvate or hydrate of the compound, the salt or the prodrug;
for the manufacture of a drug allowing neuroprotection for the treatment and/or prophylaxis of neurological diseases and conditions.

3. Use according to claim 1 or 2, where the neurological disease or condition is selected from epilepsy, dementias (including dementias of the Alzheimer's type, vascular dementias, AIDS-dementia complex, etc...), parkinsonism and movement disorders (including Huntington's disease, dystonias, Gilles de la Tourette syndrome, dyskinesias etc...), motor neuron disease or amyotrophic lateral sclerosis (ALS), other neurodegenerative and/or hereditary disorders of the nervous system (including hereditary cerebellar ataxias and spinal muscular atrophies), disorders of the peripheral nervous system, including trigeminal neuralgia and peripheral neuropathies, multiple sclerosis and other demyelinating diseases of the nervous system, infantile cerebral palsy, spasticity, hemiplegia and hemiparesis, cerebrovascular disorders (including brain ischemia, stroke, transient ischemic attacks, atherosclerosis, etc...), headache, migraine, myoneural disorders (myasthenia gravis, acute muscle spasms, myopathies, etc...), disorders of the eye and visual pathways, intracranial trauma/injury, trauma/injury to nerves and spinal cord, poisoning and toxic effects of non medicinal substances, accidental poisoning by drugs medicinal substances and biologicals, neurological and psychiatric adverse effects of drugs, medicinal and biological substances (including medication-induced movement disorders), disturbance of sphincter control and sexual function, mental disorders usually diagnosed in infancy, childhood or adolescence (including, attention deficit and disruptive behaviour disorders, autism, TIC disorders, etc ...), delirium and other cognitive disorders, substance related disorders (alcohol, nicotine, drugs), schizophrenia and other psychotic disorders, mood disorders (including depressive disorders and bipolar disorders), anxiety disorders, sexual disorders, eating disorders, sleep disorders, endocrine and metabolic diseases (diabetes, hypoglycaemia), acute and chronic pain; nausea and vomiting; irritable bowel syndrome.

4. A pharmaceutical compositions comprising a pharmaceutically effective amount of a compound of formula (I), a pharmaceutically acceptable salt thereof, a prodrug of the compound or the salt, or a solvate or hydrate of the compound, the salt or the prodrug, in combination with a pharmaceutically acceptable carrier, diluent or excipient.

5. A pharmaceutical composition comprising a pharmaceutically effective amount of a salt of formula (II), a prodrug thereof, or a solvate or hydrate of the salt or the prodrug, in combination with a pharmaceutically acceptable carrier, diluent or excipient.

6. A pharmaceutical composition according to claim 4 or 5 for the treatment of a neurological disease or condition selected from epilepsy, dementias (including dementias of the Alzheimer's type, vascular dementias, AIDS-dementia complex, etc...), parkinsonism and movement disorders (including Huntington's disease, dystonias, Gilles de la Tourette syndrome, dyskinesias etc...), motor neuron disease or amyotrophic lateral sclerosis (ALS), other neurodegenerative and/or hereditary disorders of the nervous system (including hereditary cerebellar ataxias and spinal muscular atrophies), disorders of the peripheral nervous system, including trigeminal neuralgia and peripheral neuropathies, multiple sclerosis and other demyelinating diseases of the nervous system, infantile cerebral palsy, spasticity, hemiplegia and hemiparesis, cerebrovascular disorders (including brain ischemia, stroke, transient ischemic attacks, atherosclerosis, etc...), headache, migraine, myoneural disorders (myasthenia gravis, acute muscle spasms, myopathies, etc...), disorders of the eye and visual pathways, intracranial trauma/injury, trauma/injury to nerves and spinal cord, poisoning and toxic effects of non medicinal substances, accidental poisoning by drugs medicinal substances and biologicals, neurological and psychiatric adverse effects of drugs, medicinal and biological substances (including medication-induced movement disorders), disturbance of sphincter control and sexual function, mental disorders usually diagnosed in infancy, childhood or adolescence (including, attention deficit and disruptive behaviour disorders, autism, TIC disorders, etc ...), delirium and other cognitive disorders, substance related disorders (alcohol, nicotine, drugs), schizophrenia and other psychotic disorders, mood disorders (including depressive disorders and bipolar disorders), anxiety disorders, sexual disorders, eating disorders, sleep disorders, endocrine and metabolic diseases (diabetes, hypoglycaemia), acute and chronic pain; nausea and vomiting; irritable bowel syndrome.
